# EUROPEAN PATENT SPECIFICATION

(11) **EP 4 243 903 B1**
(45) Date of publication and mention of the grant of the patent: **21.08.2024**
(21) Application number: 21810352.1
(22) Date of filing: 12.11.2021
(51) Int. Cl.: A61M 16/00

(54) **LUNG VOLUME RECRUITMENT (LVR) AUTOMATIC MANEUVER**
AUTOMATISCHES MANÖVER ZUR LUNGENVOLUMENREKRUTIERUNG (LVR)
MANOEUVRE AUTOMATIQUE DE RECRUTEMENT DE VOLUME PULMONAIRE (RVP)

(30) Priority: 13.11.2020 US 202063113232 P
(43) Date of publication of application: 20.09.2023
(73) Proprietor: Koninklijke Philips N.V., 5656 AG Eindhoven (NL)
(72) Inventor: TROXELL, David, Aaron, 5656 AG Eindhoven (NL); LEE, Seunghyun, 5656 AG Eindhoven (NL); NILSESTUEN, Jon, 5656 AG Eindhoven (NL)
(74) Representative: Philips Intellectual Property & Standards
(86) International application number: PCT/EP2021/081464
(87) International publication number: WO 2022/101381

(56) References cited:
- US-A1- 2009 197 240
- KISS THOMAS ET AL: "Lung recruitment techniques in the ICU Chapter: Lung recruitment techniques in the ICU Oxford Textbook of Critical Care (2 ed.) Lung recruitment techniques in the ICU", 1 April 2016 (2016-04-01), pages 1 - 16, XP055890651, ISBN: 978-0-19-960083-0, Retrieved from the Internet <URL:https://oxfordmedicine.com/view/10.1093/med/9780199600830.001.0001/med-9780199600830-chapter-120?print=pdf> [retrieved on 20220211], DOI: 10.1093/med/9780199600830.003.0120
- ARNAL JEAN-MICHEL ET AL: "Optimal duration of a sustained inflation recruitment maneuver in ARDS patients", INTENSIVE CARE MEDICINE, SPRINGER BERLIN HEIDELBERG, BERLIN/HEIDELBERG, vol. 37, no. 10, 20 August 2011 (2011-08-20), XP037136357, ISSN: 0342-4642, [retrieved on 20110820], DOI: 10.1007/S00134-011-2323-0
- BECHER T ET AL: "Global and regional assessment of sustained inflation pressure-volume curves in patients with acute respiratory distress syndrome", PHYSIOLOGICAL MEASUREMENT, INSTITUTE OF PHYSICS PUBLISHING, BRISTOL, GB, vol. 38, no. 6, 22 May 2017 (2017-05-22), pages 1132 - 1144, XP020316787, ISSN: 0967-3334, [retrieved on 20170522], DOI: 10.1088/1361-6579/AA6923

## Description

### CROSS-REFERENCE TO RELATED APPLICATIONS

This patent application claims the priority benefit under 35 U.S.C. § 119(e) of U.S. Provisional Application No. 63/113,232, filed on November 13, 2020.

The following relates generally to the ventilation therapy arts, and in particular to Lung Volume Recruitment (LVR) therapy, LVR as part of mechanical insufflation-exsufflation (MI-E) therapy, and related arts.

### BACKGROUND

Neuromuscular disease (NMD) refers to a group of diseases (i.e., motor neuron diseases, muscular dystrophies, and so forth) that are collectively associated with progressive respiratory muscle weakness that ultimately result in a decline in vital capacity, a decrease in chest wall and lung compliance, atelectasis, an increase in work of breathing, and an impaired ability to cough. As a result, progressive neuromuscular diseases carry an increased risk of respiratory infection, respiratory failure, and mortality (see, e.g., Ambrosino N, Carpene N, Gherardi M: Chronic respiratory care for neuromuscular diseases in adults. Eur Respir J 2009; 34: 444-451.). Non-invasive ventilation (NIV), mechanical insufflation-exsufflation (MI-E), and lung volume recruitment (LVR) are some examples of respiratory support strategies for patients with NMD (see, e.g., Simonds, A: Recent Advances in Respiratory Care for Neuromuscular Disease. Chest 2006; 130: 1879-1886; McKim D, Katz S, Barrowman N, Ni A, LeBlanc C: Lung Volume Recruitment Slows Pulmonary Function Decline in Duchenne Muscular Dystrophy. Arch Phys Med Rehabil 2012; 93: 1117-1121).

Lung Volume Recruitment (LVR) is an important therapeutic intervention; that when properly administrated, is associated with the mitigation and or reversal of alveolar atelectasis, improvement in chest wall compliance, and aids in the assisted cough effort aimed at avoiding respiratory infections (see e.g., Kiss Thomas ET AL: "Lung recruitment techniques in the ICU Chapter: Lung recruitment techniques in the ICU Oxford Textbook of Critical Gare (2 ed.) Lung recruitment techniques in the ICU", 1 April 2016 (2016-04-01), pages 1-16, XP055890651). In the LVR technique, the lungs are inflated by way of an externally applied airway pressure to increase lung volume. The term "recruitment" thus refers to the recruitment of additional lung volume by way of the LVR therapy. LVR can be separated into two primary application techniques. The more commonly known technique is often termed "breath-stacking" associated with volume delivery whereas the second technique utilizes a pressure control mode. Breath-stacking may be delivered in its most simple construct utilizing a resuscitation bag combined with an integrated one-way valve. The integration of a one-way valve in the resuscitation bag prevents the patient from exhaling while facilitating stacking of successive inhalation breaths; one on top of the other, thereby increasing the net lung volume achieved. The resuscitation bag is inexpensive, and the technique is relatively simple since the addition of the one-way valve means the patient does not need to have glottis control in order to prevent exhalation during the delivery of the successive inhalation breaths. This technique does require that the patient have upper or lower limb mobility to squeeze the bag if performed autonomously, otherwise caregiver assistance and training on an effective technique would be indicated. LVR therapy by way of a ventilator operated in pressure control mode requires more complex and expensive equipment.

Mechanical insufflation-exsufflation (MI-E) therapy is a technique in which positive airway pressure is delivered to the lungs during the inhalation phase to build up a large tidal volume, followed by application of negative airway pressure during the exhalation phase. MI-E therapy is designed to clear the lungs of secretions by producing high expiratory flow rates and is often prescribed as part of home therapy for NMD patients (see e.g. ARNAL JEAN-MICHEL ET AL: "Optimal duration of a sustained inflation recruitment maneuver in ARDS patients", INTENSIVE GARE MEDICINE, SPRINGER BERLIN HEIDELBERG, BERLIN/HEIDELBERG, vol. 37, no. 10, 20 August 2011 (2011-08-20), XP037136357). Patients undergoing MI-E therapy would often also benefit from LVR therapy. A resuscitation bag is typically used due to its low cost and suitability for unsupervised home therapy. Thus, a home therapy device combining MI-E and LVR requires that the patient switch between the MI-E device and the resuscitation bag to perform the LVR.

US patent application n°2009/197240 discloses methods and systems of maintaining, evaluating, and providing therapy to a lung ex vivo. The methods and systems involve positioning the lung in an ex vivo perfusion circuit; circulating a perfusion fluid through the lung, the fluid entering the lung through a pulmonary artery interface and leaving the lung through a left atrial interface; and ventilating the lung by flowing a ventilation gas through a tracheal interface. Maintaining the lung for extended periods involves causing the lung to rebreath a captive volume of air, and reaching an equilibrium state between the perfusion fluid and the ventilation gas. Evaluating the gas exchange capability of the lung involves deoxygenating the perfusion fluid and measuring a time taken to reoxygenate the perfusion fluid by ventilating the lung with an oxygenation gas.

The following discloses certain improvements.

### SUMMARY

In one aspect, a mechanical ventilation system includes a mechanical ventilator configured to deliver ventilation to a patient. An electronic controller is programmed to control the mechanical ventilator to perform a LVR therapy method. The LVR therapy method includes at least one LVR cycle including: an inspiration phase in which air is delivered to an upper airway of the patient by the mechanical ventilator to ramp an airway pressure up to an LVR pressure of the LVR cycle, a hold phase in which the airway pressure is maintained by the mechanical ventilator at the LVR pressure or at a pressure above the LVR pressure for a hold time interval, and an expiration phase in which the airway pressure decreases to a positive end-expiratory pressure (PEEP) of the LVR cycle.

In another aspect, a non-transitory computer readable medium stores instructions executable by an electronic controller of a mechanical ventilator to perform a LVR therapy method in which the LVR therapy method comprising a plurality of LVR cycles. Each LVR cycle includes: an inspiration phase in which air is delivered to an upper airway of the patient by the mechanical ventilator to ramp an airway pressure up to an LVR pressure of the LVR cycle, a hold phase in which the airway pressure is maintained by the mechanical ventilator at the LVR pressure or at a pressure above the LVR pressure for a hold time interval, and an expiration phase in which the airway pressure decreases to a PEEP of the LVR cycle.

In another aspect, there is disclosed but not claimed a LVR therapy method, in which a non-invasive patient accessory is coupled to a mechanical ventilator configured to deliver ventilation to a patient to an upper airway of the patient. The LVR therapy method includes a plurality of LVR cycles including: delivering air to an upper airway of the patient by the mechanical ventilator to ramp an airway pressure up to an LVR pressure of the LVR cycle; maintaining the airway pressure with the mechanical ventilator at the LVR pressure or at a pressure above the LVR pressure for a hold time interval; decreasing the airway pressure to a PEEP of the LVR cycle; for each LVR cycle, determining a lung recruitment volume as a highest lung volume measured during the LVR cycle; and terminating the LVR therapy method in response to the lung recruitment volume for a most recently completed LVR cycle being no larger than the lung recruitment volume of a preceding LVR cycle.

One advantage resides in providing a respiratory therapy device including a mechanical ventilator to perform LVR therapy without requiring the user to be capable of squeezing a resuscitation bag.

Another advantage resides in providing a mechanical ventilator without a complex device setup, configurations, or patient-specific titration settings.

Another advantage resides in providing a mechanical ventilator for providing LVR that is easier for a clinician or caregiver to operate, and/or which is suitable for use by an NMD patient in home therapy.

Another advantage resides in providing a mechanical ventilator to provide both MI-E therapy and LVR therapy for a patient, optionally automatically without user intervention.

A given embodiment may provide none, one, two, more, or all of the foregoing advantages, and/or may provide other advantages as will become apparent to one of ordinary skill in the art upon reading and understanding the present disclosure.

### BRIEF DESCRIPTION OF THE DRAWINGS

The disclosure may take form in various components and arrangements of components, and in various steps and arrangements of steps. The drawings are only for purposes of illustrating the preferred embodiments and are not to be construed as limiting the disclosure.
FIGURE 1 diagrammatically shows an illustrative apparatus for ventilator systems in accordance with the present disclosure.
FIGURE 2 shows an example flow chart of operations suitably performed by the system of FIGURE 1.
FIGURES 3 and 4 show a pressure-volume curve for a patient undergoing the operations of FIGURE 2.
FIGURE 5 shows another example flow chart of operations suitably performed by the system of FIGURE 1.
FIGURES 6, 7, 8, and 9 illustrate various automatically performed sequences of LVR therapy and MI-E therapy.

### DETAILED DESCRIPTION

As used herein, the singular form of "a", "an", and "the" include plural references unless the context clearly dictates otherwise. As used herein, statements that two or more parts or components are "coupled," "connected," or "engaged" shall mean that the parts are joined, operate, or co-act together either directly or indirectly, i.e., through one or more intermediate parts or components, so long as a link occurs. Directional phrases used herein, such as, for example and without limitation, top, bottom, left, right, upper, lower, front, back, and derivatives thereof, relate to the orientation of the elements shown in the drawings and are not limiting upon the scope of the claimed invention unless expressly recited therein. The word "comprising" or "including" does not exclude the presence of elements or steps other than those described herein and/or listed in a claim. In a device comprised of several means, several of these means may be embodied by one and the same item of hardware.

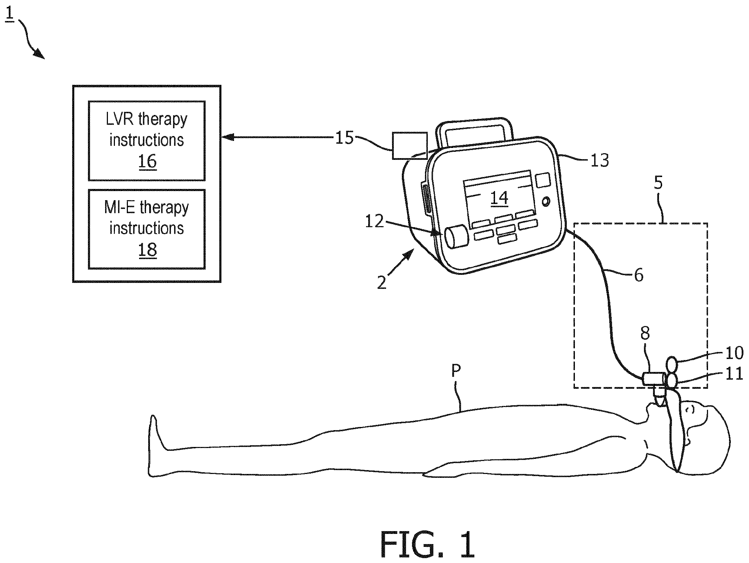

With reference to FIGURE 1, a mechanical ventilator system **1** for providing ventilation therapy to an associated patient **P** is shown. As shown in FIGURE 1, the system **1** includes a mechanical ventilator **2** configured to perform LVR therapy. For example, the mechanical ventilator **2** may be a CoughAssist airway clearance device (available from Koninklijke Philips N.V.), or a continuous positive airway pressure (CPAP) device, a Bi-Level Positive Airway Pressure (BiPAP) device, or a mechanical ventilator configured to deliver both positive airway pressure and negative airway pressure. The mechanical ventilator **2** includes an outlet (not shown) connected to a patient breathing circuit **5** to deliver mechanical ventilation to the patient **P** to perform LVR therapy (and, in some embodiments, also MI-E therapy). The patient circuit **5** includes as an air hose **6,** a non-invasive patient accessory **8** and one or more breathing sensors, such as an airflow sensor or meter **10,** an airway pressure sensor or meter **11,** and/or so forth. The illustrative sensors **10, 11** are disposed with or integrated into the non-invasive patient accessory **8;** alternatively, some or all of the sensors **10, 11** may be internal to the mechanical ventilator **2.** The non-invasive patient accessory **8** can be variously embodied, for example as a mask strapped to the patient's face. Alternatively, the non-invasive patient accessory **8** may be replaced by an invasive patient accessory such as an endotracheal tube, tracheostomy tube, or so forth. The non-invasive patient accessory **8** is configured to couple the mechanical ventilator **2** to the upper airway of the patient during the performance of an LVR therapy method **100.** As a consequence, air flow between the ventilator **2** and the lungs of the patient **P** pass through the upper airway of the patient, which includes anatomical structures such as the oropharynx, hypopharynx, larynx, and so forth.

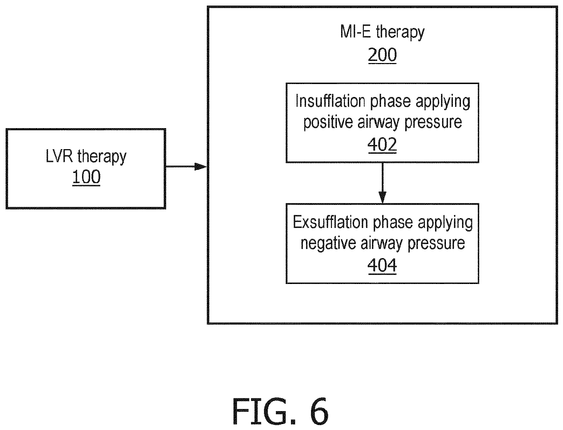

The mechanical ventilator **2** includes an air inlet (not shown) to draw atmospheric air which is delivered to the air hose **6** to provide the LVR therapy to the patient. For example, the ventilator **2** may include a blower **12** to deliver air to the air hose **6.** (Note, the blower **12** is indicated diagrammatically in FIGURE 1, as it is disposed inside the housing of the mechanical ventilator **2** and hence occluded from view). The mechanical ventilator **2** includes an electronic processor or electronic controller **13,** a display **14,** and a non-transitory computer readable medium **15** storing LVR therapy instructions **16** executable by the electronic controller **13** to perform a lung volume recruitment (LVR) therapy method **100,** and in some examples additionally MI-E instructions **18** executable by the electronic controller **13** to perform a mechanical insufflation-exsufflation (MI-E) therapy method **200,** as described in more detail with reference to FIGURE 6-9.

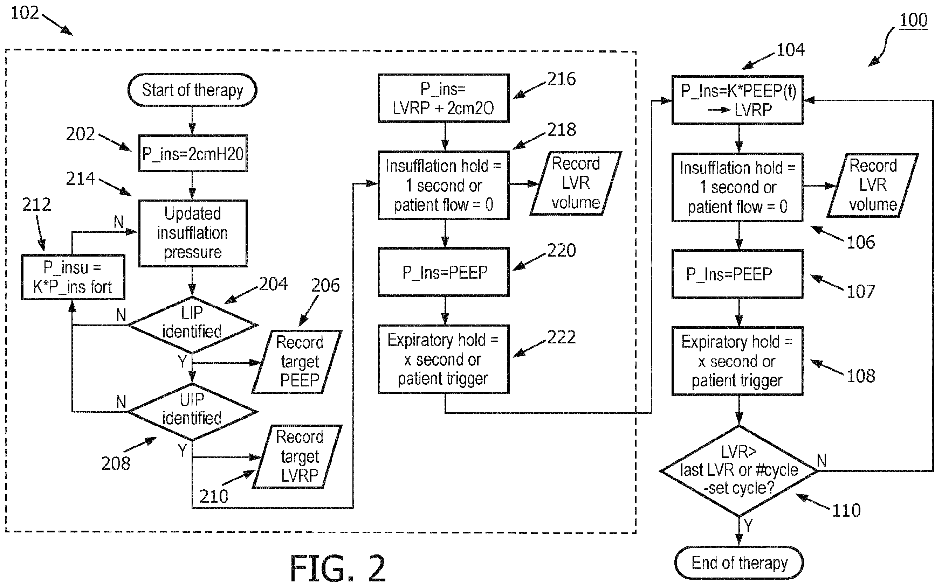

With reference to FIGURE 2, and with continuing reference to FIGURE 1, an example embodiment of the LVR therapy method **100** performed by execution of the LVR therapy instructions **16** is diagrammatically shown as a flow chart. The method **100** can be performed with a single LVR cycle, or with a plurality of LVR cycles.

To begin the method **100,** a first cycle of the LVR therapy is performed. At an operation **102,** the mechanical ventilator **2** is calibrated during a calibration LVR cycle to determine an airway pressure at which to deliver air to the patient **P.** The calibration operation **102** includes operations **202-222.** At an operation **202,** ventilation therapy is delivered by the mechanical ventilator **2** to the patient **P** with a therapy setting. The therapy setting can be a target inspiratory pressure, which can be set to maximum value tolerated by the patient (e.g., 40 cmH₂O).

At an operation **204,** a lower inflection point (LIP) of the lung-volume-versus-airway pressure curve measured during a ramp of the calibration LVR cycle during delivery of the ventilation therapy is detected. The LIP is indicative of an initial alveolar-bronchiole opening in the lungs of the patient **P.** At an operation **206,** a positive end-expiratory pressure (PEEP) value is set for an LVR cycle performed subsequent to the calibration LVR cycle based on the LIP. The PEEP value can be recorded at and stored in the non-transitory computer medium **15.**

At an operation **208,** an upper inflection point (UIP) of a lung volume-versus-airway pressure curve measured during a ramp during delivery of the ventilation therapy of the calibration LVR cycle is detected. The UIP is indicative of a recruitment pressure ceiling or upper pressure limit threshold aimed at mitigating alveolar over-distention and resulting barotrauma in the lungs of the patient **P.** At an operation **210,** an LVR pressure (LVRP) value of at least one LVR cycle performed subsequent to the calibration LVR cycle is set based on the UIP detected in the calibration LVR cycle. The LVRP value can be recorded at and stored in the non-transitory computer medium **15.**

To identify the LIP and the UIP, the calibration operation **102** implements a positive airway pressure ramp by way of an operation **212** and an operation **214,** in which the pressure of the delivered therapy is increased by a predetermined constant value *k* for a predetermined time *t* to implement the ramp. The calibration operation **102** continues until both the LIP and the UIP are detected.

At an optional operation **216,** the airway pressure of the air delivered to the patient **P** by the mechanical ventilator **2** is increased by a small incremental positive pressure above the LVRP (e.g. 2 cmH2O). The operations **202-216** thus correspond to the inspiration phase of this calibration LVR cycle. At an operation **218,** a hold phase occurs, in which the airway pressure is maintained by the mechanical ventilator **2** at the LVR pressure (or at a pressure above the LVR pressure, due to the optional operation **216)** for a hold time interval. The hold time interval during the hold phase of the LVR cycle can be determined based on detecting an airway flow decreasing to zero, or can be for a predetermined time period (e.g., three seconds). The LVR volume during this hold phase can be recorded and stored in in the non-transitory computer medium **15.** At an operation **220,** the exhalation phase of the LVR cycle begins as the patient **P** exhales to a pressure level at the recorded PEEP level, thus ending the exhalation phase. This decrease can be done passively, or in some examples controlled by the air delivered to the patient by the mechanical ventilator **2.**

At an operation **222,** airway pressure is applied by the ventilator **2** to hold the airway pressure at the PEEP of the LVR cycle, until the inhalation phase of the next LVR cycle is triggered. The next phase can be triggered in various ways, e.g. by detecting respiratory effort by the patient as an airflow change, or by the patient triggering the next LVR cycle using a handheld button or so forth.

Once the calibration operation **102** is complete, the LVR method **100** proceeds to an operation **104,** which includes an inspiration phase of a next LVR cycle is performed, in which air is delivered to an upper airway of the patient **P** by the mechanical ventilator **2** to ramp an airway pressure up to the LVRP of the LVR cycle. For example, the inspiratory airway pressure can start at the PEEP value (from the operation **220)** and can be gradually ramped or increase by the predetermined constant *k* until the inspiratory pressure approaches the recorded LVRP pressure.

At an operation **106,** similarly to the operation **218,** a hold phase occurs, in which the airway pressure is maintained by the mechanical ventilator **2** at the LVR pressure or at a pressure above the LVR pressure for a hold time interval. The hold time interval during the hold phase of the LVR cycle can again be determined based on detecting an airway flow decreasing to zero, or for a predetermined time period (e.g., three seconds). The LVR volume during this hold phase can be recorded and stored in in the non-transitory computer medium **15.** At an operation **107,** in the expiration phase the patient **P** exhales to a pressure level at the recorded PEEP level at which point the expiration phase of the LVR cycle is complete.

At an operation **108,** similar to the operation **222,** the ventilator **2** is operated to hold the PEEP until the triggering of the next LVR cycle. Upon triggering of the next LVR cycle, flow passes back to the operation **104** to initiate the inspiration phase of the next LVR cycle.

The termination of the LVR therapy **100** can be done in various ways. In one example, at an operation **110,** the electric controller **13** determines, for each LVR cycle, the LVR volume as a highest lung volume measured during the LVR cycle. If the most recently completed LVR cycle results in a LVR volume value being no larger than the LVR volume of a preceding LVR cycle, then the LVR therapy method **100** is terminated. In another example, the electronic controller **13** performs a predetermined number of LVR cycles for the LVR therapy session.

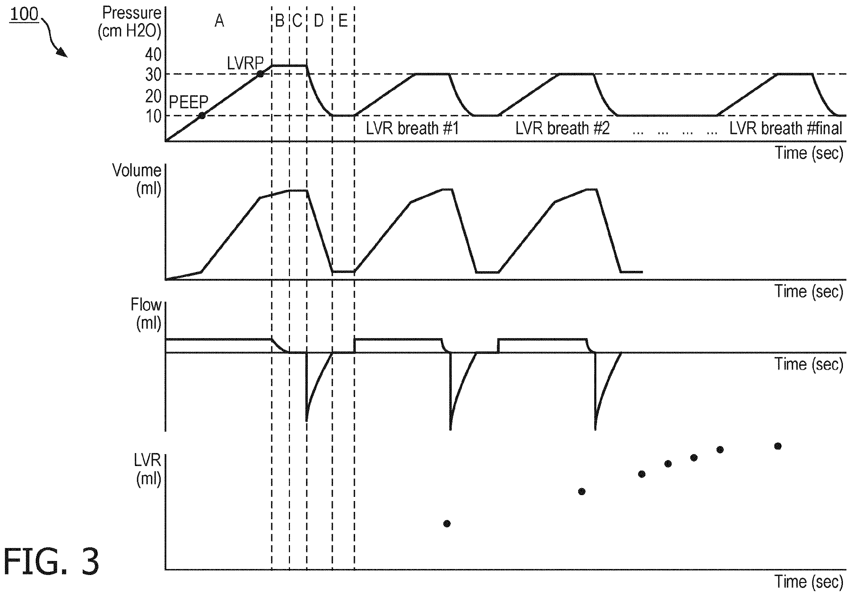

FIGURE 3 shows an example of diagrammatic plots of the airway pressure, lung volume, airway flow, and the obtained maximum lung volume. These diagrammatic plots are shown with aligned time axes (the abscissa of each plot). The airway pressure is the top plot, whose ordinate axis is labeled "Pressure {cmH2O}". The lung volume plot is the next plot down, whose ordinate axis is labeled "Volume {ml}". The airway flow is the next plot down, whose ordinate axis is labeled "Flow {lpm}". The maximum obtained lung volume (also referred to herein as the lung recruitment volume) is the bottommost plot, whose ordinate axis is labeled "LVR {ml}". The lung recruitment volume (bottommost plot) for a LVR cycle is the highest lung volume measured during the LVR cycle, and will generally occur during the hold period between the end of the inspiration phase and the start of the expiration phase of any given LVR cycle of the LVR therapy method **100.** The first LVR cycle shown in FIGURE 3 is divided into five time intervals labeled "A", "B", "C", "D", and "E". The time interval **A** is the inspiration phase in which air is delivered to the upper airway of the patient by the mechanical ventilator **2** (via the noninvasive patient accessory **8)** to ramp an airway pressure up to an LVR pressure of the LVR cycle. The time intervals **B** and **C** correspond to the hold phase of the LVR cycle, where time interval **B** is a fixed or controlled time interval and time interval **C** is a fixed inspiratory pause. The time interval **D** is the expiratory phase in which the airway pressure decreases to a PEEP of the LVR cycle. Finally, the time interval **E** is the time over which the mechanical ventilator **2** is operated to hold the airway pressure at the PEEP until the next LVR cycle commences. An optimum LVR pressure is automatically determined based on the changes in the lung compliance of the patient **P** as incremental positive pressure is applied to the patient. The patient **P** is held at the optimum LVR pressure level until the transpulmonary pressure gradient reaches zero and no further lung recruitment is achieved. The patient **P** exhales to an optimum positive end-expiratory pressure to maintain lung recruitment. The LVR therapy continues until the maximum cycle-by-cycle lung recruitment volume is achieved.

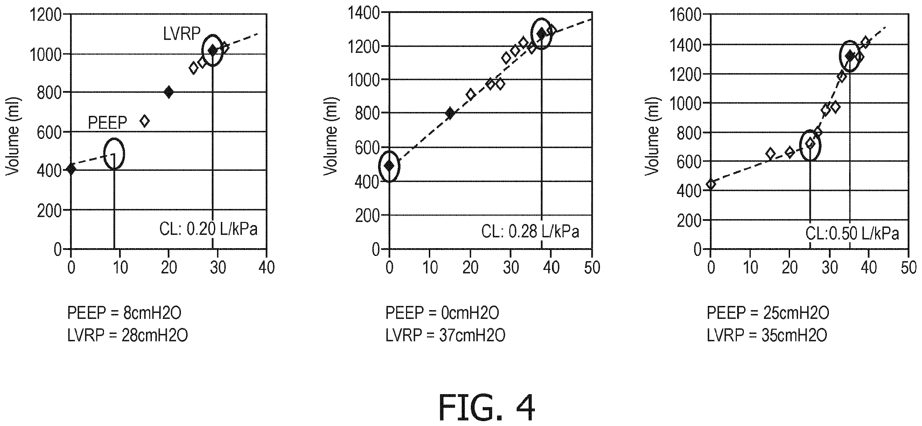

As shown in FIGURE 3, in the time interval **A** of the calibration LVR which corresponds to the inspiratory phase, a pressure ramp occurs at 10 cmH₂O/sec to identify the optimum PEEP value and the optimum LVRP value based on the LIP and the UIP from the PV curve. This corresponds to the operations **204, 206, 208, 210** of the calibration LVR cycle of the LVR therapy method **100** of FIGURE 2. FIGURE 4 shows three examples of the PV curve on NMD patients using Intermittent Positive Pressure Breathing (IPPB) or Lung Insufflation Assist Maneuver (LIAM) to titrate the optimum lung insufflation pressure or volume to achieve the maximum peak cough flow (PCF) are shown. The optimum PEEP and LVRP can be identified from each curve. (In subsequent LVR cycles, the time interval **A** may use values of PEEP and LVRP determined in the calibration LVR cycle, and hence the time interval **A** of these subsequent LVR cycles corresponds to the operation **104** of FIGURE 3).

Referring back to FIGURE 3, at the time interval **B,** the inspiratory pressure is held at the LVRP value, or at the LVRP value plus a predetermined increment (e.g., 2 cmH₂O). The hold period **B** can be a fixed time, or in another embodiment can be determined based on the flow returning to zero. This corresponds to the operation **216** of the calibration LVR cycle of FIGURE 2, or to the operation **106** for subsequent LVR cycles.

At the time interval **C,** an (optional) inspiratory pause occurs at a predetermined time interval (e.g., one second). This corresponds to the operation **222** of the calibration LVR cycle or to the operation **106** of the subsequent LVR cycles.

At the time interval **D,** the expiratory phase occurs with a passive patient exhalation towards the PEEP pressure level. This corresponds to the operation **220** of the calibration LVR cycle or to the operation **107** of the subsequent LVR cycles. In a variant embodiment, the ventilator **2** ramps the airway pressure down to control the rate of exhalation during the expiratory phase **D.**

At the time interval **E,** the mechanical ventilator 2 operates to hold the PEEP pressure until the next LVR cycle commences. an expiratory pause occurs for an arbitrary amount of time, or until the patient **P** produces an inspiratory pressure. This corresponds to the operation **222** of the calibration LVR cycle or to the operation **108** of the subsequent LVR cycles.

Referring particularly to the lowermost plot of FIGURE 3 diagrammatically plotting the lung recruitment volume for each LVR cycle, it is seen that for each successive LVR cycle the lung recruitment volume increases. This is the desired result of LVR therapy as it corresponds to lung volume recruitment. At the time marked "F" in FIGURE 3, a final LVR breath is determined based on a cycle-by-cycle LVR improvement as the lung volume recruitment plateaus. That is, the LVR therapy method **100** terminates in response to the lung recruitment volume for a most recently completed LVR cycle (corresponding to time **F)** being no larger than the lung recruitment volume of a preceding LVR cycle. This corresponds to the operation **110.** Alternatively, the LVR therapy method **100** may terminate after a predetermined, i.e. fixed, number of breath cycles (e.g., 5 cycles).

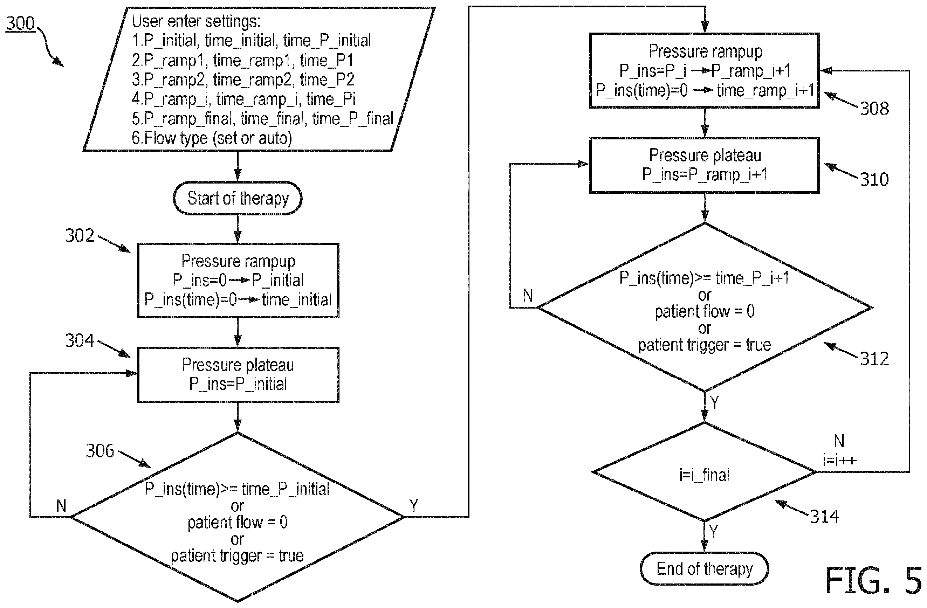

FIGURE 5 shows another embodiment of the LVR therapy method **300.** To begin the method **300,** a medical professional can enter one or more settings into the mechanical ventilator **2** via a user input device of the mechanical ventilator (now shown in FIGURE 1, but can comprise one or more buttons, one or more knobs, finger taps on the display **14,** and so forth). These settings can include, for example: a target inhale pressure setting (e.g., P_ramp_final); a ramp length (e.g., P_ramp1, P_ramp2, etc.); a number of successive LVR breaths to reach the target inhale pressure setting; a number of LVR sessions to reach target inhale pressure setting; a target inhale time setting (e.g., time_P_final); a starting inhale time setting (time_P_initial); and a patient air flow setting.

At an operation **302,** an inspiratory pressure inspiration phase occurs in which air is delivered to an upper airway of the patient **P** by the mechanical ventilator **2** to ramp an airway pressure up to the P_initial value entered by the medical professional. This ramp up occurs for a predetermined time entered by the medical professional (e.g., time_P_initial).

At an operation **304,** the airway pressure plateaus when delivered airway pressure reaches the P_initial value.

At an operation **306,** the electronic controller **13** determines whether one or more predetermined conditions exist for a subsequent pressure ramp. These conditions can include determining whether a current therapy time exceeds the predetermined time entered by the medical professional (e.g., time_P_initial); whether the air flow to the patient **P** is zero; or whether a patient trigger occurs. The operation **304** is repeated until one of these conditions is satisfied.

At an operation **308,** a subsequent ramp phase of the airway pressure is performed (e.g., P_ramp1) for a predetermined time (e.g., time_ramp1). At operations **310** and **312,** the plateau operation and the predetermined conditions operation are repeated for the subsequent ramp phase.

At an operation **314,** the electronic controller determines whether the number of ramp phase iterations reaches a final iteration entered by the medical professional (e.g., time_P_ramp _final). If the final iteration is reached, the LVR therapy method 300 ends. If the final iteration is not reached, the operations **308, 310, 312, 314** are repeated until the final iteration is reached.

The disclosed LVR method **100** advantageously delivers effective, automated lung volume recruitment maneuvers solely using inputs from a combination of pressure and flow signals, for example by identification of a zero-flow point as compared to a volume measurement, and provides a streamlined LVR approach suitable for both the hospital and for home environments where there may be no skilled clinician to deliver the LVR maneuver. The disclosed LVR method **100** is suitably employed in conjunction with a noninvasive patient accessory **8,** which as a mask. This again facilitates home therapy and avoids a surgical procedure such as a tracheotomy. Because the LVR method **100** includes measuring airway pressure and airway flow (and hence lung volume), accurate leak estimation can be integrated into the LVR method **100** to determine whether an effective or successful automated lung volume recruitment maneuver was delivered to the patient, and can be used to initiate a feedback loop when adjustments in technique or mask seal were indicated (e.g., the mechanical ventilator **2** may inform the patient **P** via a display and/or synthesized voice to "reposition your facemask"). For example, leak detection can be done by detecting the total volume of air nominally inhaled during the inspiration phase (time interval **A)** is greater than the volume of air nominally exhaled during the expiration phase (time interval **D).** Another approach for leak detection is to detect that the airflow never goes to zero during the hold time interval **B.** For example, if the hold time interval **B** is terminated when the airway flow goes to zero, then if this does not occur within some predetermined time then the expiration phase is performed, and the patient is asked to adjust the mask seal.

Because the LVR method **100** is delivered via the upper airway of the patient **P,** there is the potential for the patient **P** to exhibit an inspiratory adduction reflex in response to the airway pressure ramp of the inspiratory phase of the LVR cycle. This can be handled in various ways. The inspiratory adduction reflex is readily detected as an interruption in the airway flow during the airway pressure ramp. In one remedial approach, the ramp may be stopped until the reflex terminates, and then continued. Alternatively, the LVR cycle may be aborted entirely in response to detection of the inspiratory adduction reflex, and the next LVR cycle may be initiated upon termination of the reflex and can employ a slower ramp (i.e. the pressure increase per unit time of the ramp is reduced) so as to reduce likelihood of inducing an inspiratory adduction reflex.

Because the disclosed LVR methods and systems are noninvasive, they can be advantageously implemented using any type of mechanical ventilator that is capable of applying positive airway pressure. For example, the mechanical ventilator **2** can be a continuous positive airway pressure (CPAP) device, a Bi-Level Positive Airway Pressure (BiPAP) device, or a mechanical ventilator configured to deliver both positive airway pressure and negative airway pressure such as a Trilogy 100 or Trilogy EVO ventilator (available from Koninklijke Philips N.V.).

In other embodiments, the mechanical ventilator **2** can be a device designed to perform mechanical insufflation-exsufflation (MI-E), such as a CoughAssist airway clearance device (available from Koninklijke Philips N.V.). In this case, the disclosed LVR therapy can be advantageously performed in conjunction with MI-E therapy.

With reference to FIGURE 6, an example of such a combined therapy is described. In this embodiment, the LVR therapy method **100** is performed preparatory to (i.e., before) - performing an MI-E therapy method **200** (e.g., suitably performed by the electronic controller **13** of the mechanical ventilator **2** executing the MI-E instructions **18** as diagrammatically shown in FIGURE 1 to perform the mechanical insufflation-exsufflation (MI-E) therapy method **200).** As diagrammatically shown in FIGURE 6, the MI-E therapy method **200** includes: an insufflation phase **402** in which a positive airway pressure is applied to the airway of the patient **P** to inflate the lungs; followed by an exsufflation phase **404** in which a negative airway pressure is applied to the airway of the patient **P** to rapidly deflate the lungs so as to generate an artificial cough to remove secretions from the lungs. It should be noted that, as is conventional in the art, the airway pressures referred to herein are gauge pressures. While the exsufflation phase **404** preferably applies a negative airway pressure, in some patients this can induce an upper airway collapse - in these cases the magnitude of the negative pressure can be reduced, or in extreme cases the exsufflation phase **404** may apply an airway pressure of zero (albeit with reduced cough simulation effectiveness). To perform both the LVR therapy method **100** followed by the MI-E therapy method **200** in sequence without a delay in-between, the same mechanical ventilator **2** is preferably used to perform both the LVR therapy method **100** and the subsequent MI-E therapy method **200.** Hence, the mechanical ventilator **2** should be capable of applying a negative airway pressure in order to perform the exsufflation phase **404** of the MI-E therapy **200.** A CPAP or BiPAP device generally cannot apply a negative airway pressure; however, the Trilogy 100 or Trilogy EVO ventilator or the CoughAssist airway clearance device (all available from Koninklijke Philips N.V. as previously noted) can apply a negative airway pressure. Hence, the Trilogy 100 or Trilogy EVO ventilator, or the CoughAssist airway clearance device, are suitable devices that can be programmed to perform the therapeutic sequence of FIGURE 6.

The therapeutic combination shown in FIGURE 6 in which an LVR therapy method **100** is performed followed by the MI-E therapy method **200** can be advantageous because the LVR therapy provides lung recruitment which can increase the volume of air that can be inspired during the insufflation phase **402,** thereby increasing the volume of air that is expelled during the following exsufflation phase **404.** This larger volume of air increases the impetus for secretion removal by the MI-E therapy.

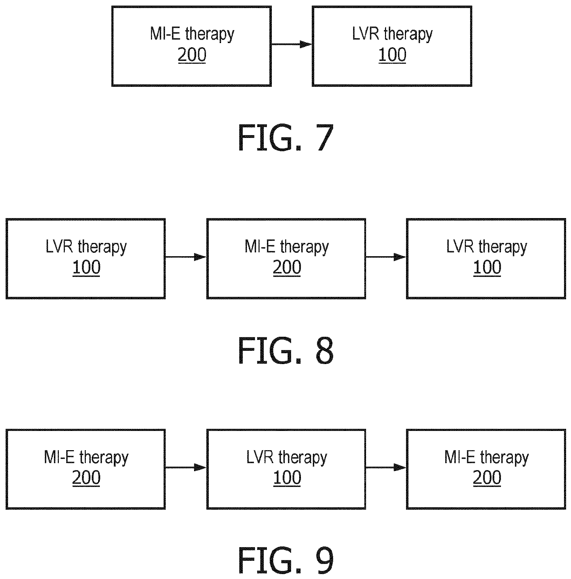

With reference to FIGURE 7, in an alternative combined therapy, the MI-E therapy method **200** is performed first, followed by the LVR therapy method **100.** This sequence of therapies can be advantageous in that during the initial MI-E therapy the application of negative pressure may result in lung de-recruitment. Therefore, an LVR therapy following MI-E therapy can restore the lung to the previous volume or even improve lung volumes and both lung and chest wall compliance.

With reference to FIGURE 8, the therapeutic sequences of FIGURES 6 and 7 can be combined so as to perform: a first instance of the LVR therapy method **100,** followed by the MI-E therapy **200,** followed by a second instance of the LVR therapy method **100.** The first instance of the LVR therapy **100** recruits additional lung volume to thereby increase effectiveness of the MI-E therapy **200,** and the secretion removal due to the MI-E therapy **200** then increases the efficacy of the second instance of the LVR therapy **100.**

With reference to FIGURE 9, an alternative combination of the therapeutic sequences of FIGURES 6 and 7 is to perform: a first instance of the MI-E therapy method **200,** followed by the LVR therapy **100,** followed by a second instance of the MI-E therapy method **200.** Here the first instance of the MI-E therapy **200** initially clears secretions from the lungs, the subsequent LVR therapy recruits additional lung volume, and the second instance of the MI-E therapy **100** removes more phlegm due to the additional lung volume driving the phlegm removal.

Advantageously, any of the therapeutic sequences of FIGURES 6-9 can optionally be performed automatically and without patient intervention. This enables synergistic combinations of LVR and MI-E therapies, such as the examples of FIGURES 6-9, to be performed in a home therapy setting without availability of a skilled clinician and without requiring the NMD patient **P** to perform difficult operations or sequences of operations. The patient **P** merely needs to maintain the seal of the mask or other noninvasive patient accessory **8** - and if this seal is compromised during the therapy this is readily detected as a leak as previously described, so that the patient can be instructed to reposition the mask or other noninvasive patient accessory **8.**

Furthermore, the LVR therapy **100** is suitable for use in a home therapy setting, either alone or in combination with MI-E therapy **200.** In a home therapy setting, the calibration LVR cycle of the LVR therapy method **100** ensures that the LVRP is automatically tuned to the specific patient **P.** The illustrative calibration cycle also tunes the PEEP of subsequent LVR cycles for the specific patient **P.** The optional tuning of the hold time during time interval **B** (see FIGURE 3) such that the airway pressure is held until the airway flow returns to zero further optimizes the LVR therapy for the specific patient **P.** The number of LVR cycles is also optionally tuned to the specific patient **P** by way of the stop criterion implemented as operation **110** of FIGURE 2. Automatic leak detection is also incorporated.

The disclosure has been described with reference to the preferred embodiments. Modifications and alterations may occur to others upon reading and understanding the preceding detailed description. It is intended that the exemplary embodiment be construed as including all such modifications and alterations insofar as they come within the scope of the appended claims which define the invention.

## Claims

1. A mechanical ventilation system **(1),** comprising:
a mechanical ventilator **(2)** configured to deliver ventilation to a patient; and
an electronic controller **(13)** programmed to control the mechanical ventilator to perform a lung volume recruitment LVR therapy method **(100),** the LVR therapy method comprising at least one LVR cycle including:
an inspiration phase **(A)** in which air is delivered to an upper airway of the patient by the mechanical ventilator to ramp an airway pressure up to an LVR pressure of the LVR cycle,
a hold phase **(B,C)** in which the airway pressure is maintained by the mechanical ventilator at the LVR pressure or at a pressure above the LVR pressure for a hold time interval, and
an expiration phase **(D)** in which the airway pressure decreases to a positive end-expiratory pressure PEEP of the LVR cycle,
**characterized in that** the electronic controller (13) is programmed to control the mechanical ventilator (2) to perform a mechanical insufflation-exsufflation (MI-E) therapy method;
wherein the electronic controller is programmed
either to perform a sequence including performing the LVR therapy method (100) and then performing the MI E therapy method in response to completion of the LVR therapy method
or to perform a sequence including performing the MI-E therapy method and then performing the LVR therapy method in response to completion of the MI-E therapy method.

2. The mechanical ventilation system (1) of claim 1, wherein the LVR therapy method includes a plurality of LVR cycles.

3. The mechanical ventilation system **(1)** of claim 2, wherein the plurality of LVR cycles includes a calibration LVR cycle that further includes:
detecting an upper inflection point UIP of a lung volume-versus-airway pressure curve measured during the ramp of the calibration LVR cycle;
wherein the LVR therapy method further includes setting the LVR pressure of at least one LVR cycle performed subsequent to the calibration LVR cycle based on the UIP detected in the calibration LVR cycle.

4. The mechanical ventilation system **(1)** of claim 3, wherein the calibration LVR cycle further includes:
detecting a lower inflection point LIP of the lung-volume-versus-airway pressure curve measured during the ramp of the calibration LVR cycle;
wherein the LVR therapy method **(100)** further includes setting the PEEP of at least one LVR cycle performed subsequent to the calibration LVR cycle based on the LIP.

5. The mechanical ventilation system **(1)** of any one of claims 2-4, wherein the LVR therapy method (100) further comprises:
for each LVR cycle, determining a lung recruitment volume as a highest lung volume measured during the LVR cycle; and
terminating the LVR therapy method in response to the lung recruitment volume for a most recently completed LVR cycle being no larger than the lung recruitment volume of a preceding LVR cycle.

6. The mechanical ventilation system **(1)** of any one of claims 1-5, wherein the performing of the LVR cycle further includes:
determining the hold time interval during the hold phase of the LVR cycle based on detecting an airway flow decreasing to zero.

7. The mechanical ventilation system **(1)** of any one of claims 1-6, further comprising:
a non-invasive patient accessory **(8)** configured to couple the mechanical ventilator **(2)** to the upper airway of the patient during the performance of the LVR therapy method **(100).**

8. The mechanical ventilation system **(1)** of any one of claims 1-7, wherein the mechanical ventilator **(2)** is a continuous positive airway pressure CPAP device, a Bi-Level Positive Airway Pressure (BiPAP) device, or a mechanical ventilator configured to deliver both positive airway pressure and negative airway pressure.

9. A non-transitory computer readable medium **(15)** storing instructions executable by an electronic controller **(15)** of a mechanical ventilator **(2)** to perform a lung volume recruitment LVR therapy method **(100),** the LVR therapy method comprising a plurality of LVR cycles, each LVR cycle including:
an inspiration phase in which air is delivered to an upper airway of the patient by the mechanical ventilator to ramp an airway pressure up to an LVR pressure of the LVR cycle,
a hold phase in which the airway pressure is maintained by the mechanical ventilator at the LVR pressure or at a pressure above the LVR pressure for a hold time interval, and
an expiration phase in which the airway pressure decreases to a positive end-expiratory pressure PEEP of the LVR cycle, **characterized in that** the instructions cause the electronic controller (15) to control the mechanical ventilator (2) to perform a mechanical insufflation-exsufflation (MI-E) therapy method; and
either to perform a sequence including performing the LVR therapy method (100) and then performing the MI E therapy method in response to completion of the LVR therapy method
or to perform a sequence including performing the MI-E therapy method and then performing the LVR therapy method in response to completion of the MI-E therapy method.

10. The non-transitory computer readable medium **(15)** of claim 9, wherein the plurality of LVR cycles includes a calibration LVR cycle that further includes:
detecting an upper inflection point UIP of a lung volume-versus-airway pressure curve measured during the ramp of the calibration LVR cycle;
wherein the LVR therapy method further includes setting the LVR pressure of at least one LVR cycle performed subsequent to the calibration LVR cycle based on the UIP detected in the calibration LVR cycle.

11. The non-transitory computer readable medium **(15)** of claim 10, wherein the calibration LVR cycle further includes:
detecting a lower inflection point LIP of the lung-volume-versus-airway pressure curve measured during the ramp of the calibration LVR cycle;
wherein the LVR therapy method **(100)** further includes setting the PEEP of at least one LVR cycle performed subsequent to the calibration LVR cycle based on the LIP.

12. The non-transitory computer readable medium **(15)** of any one of claims 10-11, wherein the LVR therapy method **(100)** further comprises:
for each LVR cycle, determining a lung recruitment volume as a highest lung volume measured during the LVR cycle; and
terminating the LVR therapy method in response to the lung recruitment volume for a most recently completed LVR cycle being no larger than the lung recruitment volume of a preceding LVR cycle.

13. The non-transitory computer readable medium (15) of any one of claims 9-12, wherein the performing of the LVR cycle further includes:
determining the hold time interval during the hold phase of the LVR cycle based on detecting an airway flow decreasing to zero.

## Patentansprüche

1. Mechanisches Beatmungssystem **(1),** umfassend:
ein mechanisches Beatmungsgerät **(2),** das für die Beatmung eines Patienten konfiguriert ist; und
eine elektronische Steuereinheit **(13),** die so programmiert ist, dass sie das mechanische Beatmungsgerät steuert, um ein Lungenvolumenrekrutierungs-, LVR-, Therapieverfahren **(100)** durchzuführen, wobei das LVR-Therapieverfahren mindestens einen LVR-Zyklus umfasst, der Folgendes beinhaltet:
eine Einatmungsphase **(A),** in der Luft durch das mechanische Beatmungsgerät in einen oberen Atemweg des Patienten zugeführt wird, um einen Atemwegsdruck auf einen LVR-Druck des LVR-Zyklus zu erhöhen,
eine Haltephase **(B, C),** in der der Atemwegsdruck durch das mechanische Beatmungsgerät für ein Haltezeitintervall auf dem LVR-Druck oder auf einem Druck oberhalb des LVR-Drucks gehalten wird, und
eine Ausatmungsphase **(D),** in der der Atemwegsdruck auf einen positiven end-exspiratorischen Druck (PEEP) des LVR-Zyklus sinkt,
**dadurch gekennzeichnet, dass** die elektronische Steuereinheit (13) so programmiert ist, dass sie das mechanische Beatmungsgerät (2) so steuert, dass es ein mechanisches Insufflations-Exsufflations- (MI-E-) Therapieverfahren durchführt;
wobei die elektronische Steuereinheit so programmiert ist,
dass sie entweder eine Sequenz durchführt, die die Durchführung des LVR-Therapieverfahrens (100) und dann die Durchführung des MI-E-Therapieverfahrens als Reaktion auf die Beendigung des LVR-Therapieverfahrens beinhaltet,
oder dass sie eine Sequenz durchführt, die die Durchführung des MI-E-Therapieverfahrens und dann die Durchführung des LVR-Therapieverfahrens als Reaktion auf die Beendigung des MI-E-Therapieverfahrens beinhaltet.

2. Mechanisches Beatmungssystem **(1)** nach Anspruch 1, wobei das LVR-Therapieverfahren eine Vielzahl von LVR-Zyklen beinhaltet.

3. Mechanisches Beatmungssystem **(1)** nach Anspruch 2, wobei die Vielzahl von LVR-Zyklen einen Kalibrierungs-LVR-Zyklus beinhaltet, der weiter Folgendes beinhaltet:
Erfassen eines oberen Wendepunktes (UIP) einer Lungenvolumen-gegenüber-Atemwegsdruck-Kurve, gemessen während des Anstiegs des LVR-Kalibrierungszyklus;
wobei das LVR-Therapieverfahren weiter das Einstellen des LVR-Drucks mindestens eines LVR-Zyklus beinhaltet, der im Anschluss an den Kalibrierungs-LVR-Zyklus auf der Grundlage des im Kalibrierungs-LVR-Zyklus erfassten UIP durchgeführt wird.

4. Mechanisches Beatmungssystem **(1)** nach Anspruch 3, wobei der Kalibrierungs-LVR-Zyklus weiter Folgendes beinhaltet:
Erfassen eines unteren Wendepunktes (LIP) der Lungenvolumen-gegenüber-Atemwegsdruck-Kurve, gemessen während des Anstiegs des LVR-Kalibrierungszyklus;
wobei das LVR-Therapieverfahren **(100)** weiter das Einstellen des PEEP von mindestens einem LVR-Zyklus beinhaltet, der im Anschluss an den Kalibrierungs-LVR-Zyklus auf der Grundlage des LIP durchgeführt wird.

5. Mechanisches Beatmungssystem **(1)** nach einem der Ansprüche 2-4, wobei das LVR-Therapieverfahren **(100)** weiter Folgendes umfasst:
für jeden LVR-Zyklus Bestimmen eines Lungenrekrutierungsvolumens als höchstes während des LVR-Zyklus gemessenes Lungenvolumen; und
Beenden des LVR-Therapieverfahrens als Reaktion darauf, dass das Lungenrekrutierungsvolumen für einen zuletzt beendeten LVR-Zyklus nicht größer ist als das Lungenrekrutierungsvolumen eines vorhergehenden LVR-Zyklus.

6. Mechanisches Beatmungssystem **(1)** nach einem der Ansprüche 1-5, wobei die Durchführung des LVR-Zyklus weiter Folgendes beinhaltet:
Bestimmen des Haltezeitintervalls während der Haltephase des LVR-Zyklus auf der Grundlage der Erfassung eines auf null abnehmenden Atemwegsflusses.

7. Mechanisches Beatmungssystem **(1)** nach einem der Ansprüche 1-6, weiter umfassend:
ein nicht-invasives Patientenzubehör **(8),** das so konfiguriert ist, dass es das mechanische Beatmungsgerät **(2)** während der Durchführung des LVR-Therapieverfahrens **(100)** an den oberen Atemweg des Patienten anschließt.

8. Mechanisches Beatmungssystem **(1)** nach einem der Ansprüche 1-7, wobei das mechanische Beatmungsgerät (2) eine Vorrichtung mit kontinuierlichem positivem Atemwegsdruck (CPAP), eine Vorrichtung mit positivem Atemwegsdruck auf zwei Ebenen (Bi-Level Positive Airway Pressure, **BiPAP)** oder ein mechanisches Beatmungsgerät ist, das so konfiguriert ist, dass es sowohl positiven Atemwegsdruck als auch negativen Atemwegsdruck liefert.

9. Nichtflüchtiges computerlesbares Medium **(15),** das Anweisungen speichert, die von einer elektronischen Steuereinheit **(15)** eines mechanischen Beatmungsgeräts **(2)** ausgeführt werden können, um ein Lungenvolumenrekrutierungs-LVR-Therapieverfahren **(100)** durchzuführen, wobei das LVR-Therapieverfahren eine Vielzahl von LVR-Zyklen umfasst, wobei jeder LVR-Zyklus Folgendes beinhaltet:
eine Einatmungsphase, in der Luft durch das mechanische Beatmungsgerät in einen oberen Atemweg des Patienten zugeführt wird, um einen Atemwegsdruck auf einen LVR-Druck des LVR-Zyklus zu erhöhen,
eine Haltephase, in der der Atemwegsdruck durch das mechanische Beatmungsgerät für ein Haltezeitintervall auf dem LVR-Druck oder auf einem Druck oberhalb des LVR-Drucks gehalten wird, und
eine Ausatmungsphase, in der der Atemwegsdruck auf einen positiven end-exspiratorischen Druck (PEEP) des LVR-Zyklus sinkt,
**dadurch gekennzeichnet, dass** die Anweisungen die elektronische Steuereinheit (15) veranlassen, das mechanische Beatmungsgerät (2) so zu steuern, dass es ein mechanisches Insufflations-Exsufflations- (MI-E-) Therapieverfahren durchführt; und
dass sie entweder eine Sequenz durchführt, die die Durchführung des LVR-Therapieverfahrens (100) und dann die Durchführung des MI-E-Therapieverfahrens als Reaktion auf die Beendigung des LVR-Therapieverfahrens beinhaltet,
oder dass sie eine Sequenz durchführt, die die Durchführung des MI-E-Therapieverfahrens und dann die Durchführung des LVR-Therapieverfahrens als Reaktion auf die Beendigung des MI-E-Therapieverfahrens beinhaltet.

10. Nichtflüchtiges computerlesbares Medium **(15)** nach Anspruch 9, wobei die Vielzahl der LVR-Zyklen einen Kalibrierungs-LVR-Zyklus beinhaltet, der weiter Folgendes beinhaltet:
Erfassen eines oberen Wendepunktes (UIP) einer Lungenvolumen-gegenüber-Atemwegsdruck-Kurve, gemessen während des Anstiegs des LVR-Kalibrierungszyklus;
wobei das LVR-Therapieverfahren weiter das Einstellen des LVR-Drucks mindestens eines LVR-Zyklus beinhaltet, der im Anschluss an den Kalibrierungs-LVR-Zyklus auf der Grundlage des im Kalibrierungs-LVR-Zyklus erfassten UIP durchgeführt wird.

11. Nichtflüchtiges computerlesbares Medium **(15)** nach Anspruch 10, wobei der Kalibrierungs-LVR-Zyklus weiter Folgendes beinhaltet:
Erfassen eines unteren Wendepunktes (LIP) der Lungenvolumen-gegenüber-Atemwegsdruck-Kurve, gemessen während des Anstiegs des LVR-Kalibrierungszyklus;
wobei das LVR-Therapieverfahren **(100)** weiter das Einstellen des PEEP von mindestens einem LVR-Zyklus beinhaltet, der im Anschluss an den Kalibrierungs-LVR-Zyklus auf der Grundlage des LIP durchgeführt wird.

12. Nichtflüchtiges computerlesbares Medium **(15)** nach einem der Ansprüche 10-11, wobei das LVR-Therapieverfahren **(100)** weiter Folgendes umfasst:
für jeden LVR-Zyklus Bestimmen eines Lungenrekrutierungsvolumens als höchstes während des LVR-Zyklus gemessenes Lungenvolumen; und
Beenden des LVR-Therapieverfahrens als Reaktion darauf, dass das Lungenrekrutierungsvolumen für einen zuletzt beendeten LVR-Zyklus nicht größer ist als das Lungenrekrutierungsvolumen eines vorhergehenden LVR-Zyklus.

13. Nichtflüchtiges computerlesbares Medium **(15)** nach einem der Ansprüche 9-12, wobei die Durchführung des LVR-Zyklus weiter Folgendes beinhaltet:
Bestimmen des Haltezeitintervalls während der Haltephase des LVR-Zyklus auf der Grundlage der Erfassung eines auf null abnehmenden Atemwegsflusses.

## Revendications

1. Système de ventilation mécanique **(1),** comprenant :
un ventilateur mécanique **(2)** configuré pour administrer une ventilation à un patient ; et
un dispositif de commande électronique **(13)** programmé pour commander le ventilateur mécanique afin d'effectuer un procédé de thérapie par recrutement de volume pulmonaire, LVR, **(100),** le procédé de thérapie par LVR comprenant au moins un cycle de LVR incluant :
une phase d'inspiration **(A)** dans laquelle de l'air est administré aux voies respiratoires supérieures du patient par le ventilateur mécanique pour augmenter la pression des voies respiratoires jusqu'à une pression de LVR du cycle de LVR,
une phase de maintien **(B, C)** dans laquelle la pression des voies respiratoires est maintenue par le ventilateur mécanique à la pression de LVR ou à une pression supérieure à la pression de LVR pendant un intervalle de temps de maintien, et
une phase d'expiration **(D)** dans laquelle la pression des voies respiratoires diminue jusqu'à une pression de fin d'expiration positive PEEP du cycle de LVR,
**caractérisé en ce que** le dispositif de commande électronique (13) est programmé pour commander le ventilateur mécanique (2) afin d'effectuer un procédé de thérapie par insufflation-exsufflation mécanique (MI-E) ;
dans lequel le dispositif de commande électronique est programmé
soit pour exécuter une séquence incluant une exécution du procédé de thérapie par LVR (100), puis une exécution du procédé de thérapie par MI-E en réponse à l'achèvement du procédé de thérapie par LVR
soit pour exécuter une séquence incluant une exécution du procédé de thérapie par MI-E, puis une exécution du procédé de thérapie par LVR en réponse à l'achèvement du procédé de thérapie par MI-E.

2. Système de ventilation mécanique **(1)** selon la revendication 1, dans lequel le procédé de thérapie par LVR inclut une pluralité de cycles de LVR.

3. Système de ventilation mécanique **(1)** selon la revendication 2, dans lequel la pluralité de cycles LVR inclut un cycle de LVR d'étalonnage qui inclut en outre :
la détection d'un point d'inflexion supérieur, UIP, d'une courbe de pression des voies respiratoires-versus-volume pulmonaire mesurée pendant l'augmentation du cycle de LVR d'étalonnage ;
dans lequel le procédé de thérapie par LVR inclut en outre un réglage de la pression de LVR d'au moins un cycle de LVR effectué après le cycle de LVR d'étalonnage sur la base de l'UIP détecté dans le cycle de LVR d'étalonnage.

4. Système de ventilation mécanique **(1)** selon la revendication 3, dans lequel le cycle de LVR d'étalonnage inclut en outre :
la détection d'un point d'inflexion inférieur LIP de la courbe de pression des voies respiratoires-versus-volume pulmonaire mesuré pendant l'augmentation du cycle de LVR d'étalonnage ;
dans lequel le procédé de thérapie par LVR **(100)** inclut en outre un réglage de la PEEP d'au moins un cycle de LVR effectué après le cycle de LVR d'étalonnage sur la base du LIP.

5. Système de ventilation mécanique **(1)** selon l'une quelconque des revendications 2-4, dans lequel le procédé de thérapie par LVR **(100)** comprend en outre :
pour chaque cycle de LVR, la détermination d'un volume de recrutement pulmonaire en tant que volume pulmonaire le plus élevé mesuré pendant le cycle de LVR ; et
l'action de mettre fin au procédé de thérapie par LVR en réponse au fait que le volume de recrutement pulmonaire pour un cycle de LVR terminé le plus récemment n'est pas supérieur au volume de recrutement pulmonaire d'un cycle de LVR précédent.

6. Système de ventilation mécanique **(1)** selon l'une quelconque des revendications 1-5, dans lequel la réalisation du cycle de LVR inclut en outre :
la détermination de l'intervalle de temps de maintien pendant la phase de maintien du cycle de LVR sur la base de la détection d'un débit des voies respiratoires diminuant jusqu'à zéro.

7. Système de ventilation mécanique **(1)** selon l'une quelconque des revendications 1-6, comprenant en outre :
un accessoire de patient non invasif **(8)** configuré pour coupler le ventilateur mécanique **(2)** aux voies respiratoires supérieures du patient pendant l'exécution du procédé de thérapie par LVR **(100).**

8. Système de ventilation mécanique **(1)** selon l'une quelconque des revendications 1-7, dans lequel le ventilateur mécanique **(2)** est un dispositif à pression positive continue des voies respiratoires, CPAP, un dispositif à pression positive des voies respiratoires à deux niveaux (BiPAP), ou un ventilateur mécanique configuré pour administrer à la fois une pression positive et une pression négative des voies respiratoires.

9. Support non transitoire lisible par ordinateur **(15)** stockant des instructions exécutables par un dispositif de commande électronique **(15)** d'un ventilateur mécanique **(2)** pour effectuer un procédé de thérapie par recrutement de volume pulmonaire, LVR, **(100),** le procédé de thérapie par LVR comprenant une pluralité de cycles de LVR, chaque cycle de LVR incluant :
une phase d'inspiration dans laquelle de l'air est administré aux voies respiratoires supérieures du patient par le ventilateur mécanique pour augmenter la pression des voies respiratoires jusqu'à une pression de LVR du cycle de LVR,
une phase de maintien dans laquelle la pression des voies respiratoires est maintenue par le ventilateur mécanique à la pression de LVR ou à une pression supérieure à la pression de LVR pendant un intervalle de temps de maintien, et
une phase d'expiration dans laquelle la pression des voies respiratoires diminue jusqu'à une pression de fin d'expiration positive PEEP du cycle de LVR,
**caractérisé en ce que** les instructions amènent le dispositif de commande électronique (15) à commander le ventilateur mécanique (2) pour exécuter un procédé de thérapie par insufflation-exsufflation mécanique (MI-E) ; et
soit à exécuter une séquence incluant une exécution du procédé de thérapie par LVR (100), puis une exécution du procédé de thérapie par MI-E en réponse à l'achèvement du procédé de thérapie par LVR
soit à exécuter une séquence incluant une exécution du procédé de thérapie par MI-E, puis une exécution du procédé de thérapie par LVR en réponse à l'achèvement du procédé de thérapie par MI-E.

10. Support non transitoire lisible par ordinateur **(15)** selon la revendication 9, dans lequel la pluralité de cycles de LVR inclut un cycle de LVR d'étalonnage qui inclut en outre :
la détection d'un point d'inflexion supérieur, UIP, d'une courbe de pression des voies respiratoires-versus-volume pulmonaire mesurée pendant l'augmentation du cycle de LVR d'étalonnage ;
dans lequel le procédé de thérapie par LVR inclut en outre un réglage de la pression de LVR d'au moins un cycle de LVR effectué après le cycle de LVR d'étalonnage sur la base de l'UIP détecté dans le cycle de LVR d'étalonnage.

11. Support non transitoire lisible par ordinateur **(15)** selon la revendication 10, dans lequel le cycle de LVR d'étalonnage inclut en outre :
la détection d'un point d'inflexion inférieur LIP de la courbe de pression des voies respiratoires-versus-volume pulmonaire mesuré pendant l'augmentation du cycle de LVR d'étalonnage ;
dans lequel le procédé de thérapie par LVR **(100)** inclut en outre un réglage de la PEEP d'au moins un cycle de LVR effectué après le cycle de LVR d'étalonnage sur la base du LIP.

12. Support non transitoire lisible par ordinateur **(15)** selon l'une quelconque des revendications 10-11, dans lequel le procédé de thérapie par LVR **(100)** comprend en outre :
pour chaque cycle de LVR, la détermination d'un volume de recrutement pulmonaire en tant que volume pulmonaire le plus élevé mesuré pendant le cycle de LVR ; et
l'action de mettre fin au procédé de thérapie par LVR en réponse au fait que le volume de recrutement pulmonaire pour un cycle de LVR terminé le plus récemment n'est pas supérieur au volume de recrutement pulmonaire d'un cycle LVR précédent.

13. Support non transitoire lisible par ordinateur **(15)** selon l'une quelconque des revendications 9-12, dans lequel l'exécution du cycle de LVR inclut en outre :
la détermination de l'intervalle de temps de maintien pendant la phase de maintien du cycle de LVR sur la base de la détection d'un débit des voies respiratoires diminuant jusqu'à zéro.
